# EUROPEAN PATENT SPECIFICATION

(11) **EP 3 902 572 B1**
(45) Date of publication and mention of the grant of the patent: **04.03.2026**
(21) Application number: 19904921.4
(22) Date of filing: 25.12.2019
(51) Int. Cl.: A61L 24/02, A61L 24/04, A61K 9/70, A61K 31/755, A61K 31/80, A61K 33/10

(54) **A BONE REGENERATION MATERIAL HAVING A COTTON-WOOL LIKE STRUCTURE FORMED OF A PLURALITY OF ELECTROSPUN FIBERS**
KNOCHENREGENERATIONSMATERIAL MIT AUS EINER VIELZAHL VON ELEKTROGESPONNENEN FASERN GEFORMTER, BAUMWOLLARTIGER STRUKTUR
MATÉRIAU DE RÉGÉNÉRATION OSSEUSE AYANT UNE STRUCTURE DE TYPE OUATE FORMÉE D'UNE PLURALITÉ DE FIBRES ÉLECTROFILÉES

(30) Priority: 25.12.2018 US 201862784746 P
(43) Date of publication of application: 03.11.2021
(73) Proprietor: Orthorebirth Co., Ltd., Yokohama-shi, Kanagawa 224-0033 (JP); Theradaptive, Inc., Frederick MD 21703 (US)
(72) Inventor: TAIRA, Hiroyuki, Yokohama-shi 224-0033 (JP); ALVAREZ, Luis, Frederick, Maryland 21704 (US)
(74) Representative: HGF
(86) International application number: PCT/US2019/068509
(87) International publication number: WO 2020/139901

(56) References cited:
- US-A1- 2010 260 673
- US-A1- 2014 037 593
- US-A1- 2015 079 147
- US-A1- 2015 087 062
- US-A1- 2018 280 569
- ZEESHAN SHEIKH ET AL: "Bone Regeneration Using Bone Morphogenetic Proteins and Various Biomaterial Carriers", MATERIALS, vol. 8, no. 4, 15 April 2015 (2015-04-15), pages 1778 - 1816, XP055416139, DOI: 10.3390/ma8041778
- HUAN ZHOU ET AL: "Fabrication aspects of PLA-CaP/PLGA-CaP composites for orthopedic applications: A review", ACTA BIOMATERIALIA, ELSEVIER, AMSTERDAM, NL, vol. 8, no. 6, 25 January 2012 (2012-01-25), pages 1999 - 2016, XP028480423, ISSN: 1742-7061, [retrieved on 20120202], DOI: 10.1016/J.ACTBIO.2012.01.031
- JIN-HYUNG SHIM ET AL: "Efficacy of rhBMP-2 loaded PCL/PLGA/-TCP guided bone regeneration membrane fabricated by 3D printing technology for reconstruction of calvaria defects in rabbit", BIOMEDICAL MATERIALS, INSTITUTE OF PHYSICS PUBLISHING, BRISTOL, GB, vol. 9, no. 6, 10 November 2014 (2014-11-10), pages 65006, XP020275398, ISSN: 1748-605X, [retrieved on 20141110], DOI: 10.1088/1748-6041/9/6/065006
- BAE ET AL.: "Efficacy of rhBMP-2 Loaded PCL/B-TCP/bdECM Scaffold Fabricated by 3D Printing Technology on Bone Regeneration", BIOMED RESEARCH INTERNATIONAL, vol. 2018, 27 February 2018 (2018-02-27), pages 1 - 12, XP055722582
- LU, LICHUN; YASZEMSKI, MICHAEL J.; MIKOS, ANTONIOS G.: "TGF-beta1 Release From Biodegradable Polymer Microparticles: Its Effects on Marrow Stromal Osteoblast Function", THE JOURNAL OF BONE AND JOINT SURGERY, vol. 83-A, no. 1, 30 November 2000 (2000-11-30), pages S82 - 91, XP009529183, ISSN: 0021-9355

## Description

### BACKGROUND OF INVENTION

### Field of the Invention

The present invention relates to bone regeneration materials, particularly to bone regeneration materials formed of biodegradable fibers comprising beta-tricalcium phosphate and a bone morphogenic protein.

### Background Art

Calcium phosphate (particularly, beta-tricalcium phosphate, β-TCP) has been widely used in bone regeneration applications because it shows osteoconductive features. The release of calcium and phosphorus ions regulates the activation of osteoblasts and osteoclasts to facilitate bone regeneration. The control of surface properties and porosity of calcium phosphate affects cell/protein adhesion and growth and regulates bone mineral formation. (Jiwoon Jeong et al., "Bioactive calcium phosphate materials and applications in bone regeneration," Biomater. Res. 23, 4 (2019) doi:10.1186/s40824-018-0149-3).

One of the Applicants of the present application developed biodegradable fibers containing β-TCP using an electrospinning process, in which a spinning solution is ejected as a thin fiber from a nozzle and pulled by the electrostatic attraction in the electric field to be deposited on a collector. Using a novel electrospinning setup, the Applicant has successfully prepared such biodegradable fibers into a cotton wool-like structure, which contain β-TCP and a biodegradable polymer. (see U.S. patent Nos. 8,853,298, and 10,092,650). The cotton wool-like structure is unique and confers several advantages: (1) it contains a large interstitial space to allow biological fluids to readily permeate into the bone graft structure, (2) it offers a large surface area to allow ready release of calcium and phosphorus from β-TCP into the biological fluids; (3) it has a flexible structure that can be made to conform to the shape of the bone repair site; and (4) it offers a large surface area to support other bioactive or bone morphogenetic factors, such as BMP-2. *In vivo* and *in vitro* evaluation of the cotton wool-like composite as bone substitute material has demonstrated its advantages in the repair of complex bone defects.

Bone morphogenetic protein-2 (BMP-2) is osteoinductive and can facilitate bone formation/regeneration. For example, Infuse^{™} Bone Graft (Medtronic) contains a manufactured bone graft material containing recombinant human BMP-2 (rhBMP-2) and is approved by the Food and Drug Administration (FDA) for use as a bone graft in sinus augmentation and localized alveolar ridge augmentation. BMP-2 is incorporated into a bone implant (INFUSE) and delivered to the site of the fracture. BMP-2 is gradually released at the site to stimulate bone formation; the growth stimulation by BMPs is localized and sustained for some weeks. If BMP-2 leaks into remote sites, adverse effects would occur. Indeed, several side effects arising from rhBMP-2 have been reported. These side effects include postoperative inflammation and associated adverse effects, ectopic bone formation, osteoclast-mediated bone resorption, and inappropriate adipogenesis. (Aaron W. James et al., "A review of the Clinical Side Effects of Bone Morphogenetic Protein-2," Tissue Eng. Part B Rev., 2016, 22(4): 284-297). In another example an implant comprises extruded PCL/PLGA fibers with 20% beta-TCP coated with rhBMP-2 (JIN-HYUNG SHIM ET AL: "Efficacy of rhBMP-2 loaded PCL/PLGA/β-TCP guided bone regeneration membrane fabricated by 3D printing technology for reconstruction of calvaria defects in rabbit", BIOMEDICAL MATERIALS, 2014, vol. 9(6): 9 pages).

Therefore, there is a need for bone graft materials that can include BMP-2 in a manner that allows gradual release of BMP-2 but would not permit leaching of this growth factor to an unintended site.

### SUMMARY OF INVENTION

The invention relates to a bone regeneration material comprising a structure of biodegradable fibers comprising beta-tricalcium phosphate (b-TCP) and a biodegradable polymer, and a bone morphogenetic protein-2 (BMP-2), wherein the BMP-2 is bound to the b-TCP, wherein the BMP-2 is a targetable BMP-2 protein fused with a b-TCP-binding peptide, and the BMP-2 is bound to the b-TCP via the b-TCP-binding peptide, wherein the b-TCP is 30-85 weight % of the structure, and wherein the biodegradable fibers comprise fibers with a thickness of about 5 micrometers to about 100 micrometers, and the biodegradable fibers are formed by electrospinning. Embodiments of the invention relate to bone regeneration materials that comprise ReBOSSIS^{®} fibers and a bone morphogenetic protein-2 (BMP-2). The BMP-2 may be a human BMP-2 or an animal (e.g., pets or livestock) BMP-2. A derivative of BMP-2 includes a BMP-2 fused with one or more β-TCP binding peptides to form a fusion protein, which will be referred to as a "targetable BMP-2" or "tBMP-2." All these different forms of BMP-2, such as human BMP-2 (including recombinant human BMP-2, rhBMP-2 and wild-type human BMP-2, wtBMP-2), animal BMP-2, and tBMP-2, may be referred to generically as "BMP-2." That is, the term "BMP-2" encompasses rhBMP-2, wtBMP-2, animal BMP-2, and tBMP-2.

ReBOSSIS^{®} has a cotton-wool like structure formed of a plurality of electrospun biodegradable fibers having a diameter of 5-100 µm and containing calcium compound particles (e.g., β-TCP particles) and biodegradable polymer such as poly(lactic acid) (PLLA) or poly(lactic-co-glycolic acid) (PLGA). The biodegradable fibers may further contain other calcium compound particles, such as silicon releasing calcium carbonate vaterite (i.e., silicon-doped vaterite, SiV). Thus, ReBOSSIS^{®} fibers comprise a biodegradable polymer (e.g., PLLA or PLGA) and β-TCP particles or β-TCP and SiV particles). As used herein, the term "calcium compound particles" may be β-TCP particles, , or a combination of β-TCP particles and SiV particles.

An electrospun biodegradable fiber of ReBOSSIS^{®} contains a large amount of these calcium compound particles (30-85 wt% or 25-65 vol%) distributed on or in the fibers. A portion of the β-TCP particles are exposed on the surface of the fibers, and the remaining portion of the β-TCP particles are buried in the fibers without being exposed outside. A bone morphogenic protein 2, *i.e.* tBMP-2, is bound to the β-TCP particles and optionally SiV particles exposed on the surface of the fiber so that the BMP-2 is captured onto the β-TCP particles and optionally SiV particles exposed on the surface of the fibers of ReBOSSIS^{®} throughout the cotton-wool like structure.

The biodegradable fiber has a diameter of about 5-100 µm, preferably about 10-60 µm, such that calcium compound particles *(i.e.* β-TCP and optionally SiV particles) having a diameter of about 1-5 µm can be distributed in the fiber and mechanical strength of the cotton wool-like structure can be maintained after implantation of ReBOSSIS^{®} at the site of a bone defect.

Preferably, bulk density of a cotton wool like structure of ReBOSSIS^{®} is about 0.01 to 0.1 g/cm³ and gaps between the fibers are about 1-50 µm so that body fluids that contain bone formation contributing cells can permeate through the gaps between the fibers and space for bone formation is secured throughout the cotton-wool like structure.

After implantation of ReBOSSIS^{®} at a bone defect site, body fluids containing mesenchymal stem cells may come into contact with the BMP-2 *(i.e.* t-BMP-2) captured on the β-TCP particles. Then, the BMP-2 *(i.e.* tBMP-2) promotes osteoprogenitor cells to differentiate into osteoblast cells. The β-TCP particles that bind BMP-2 are gradually dissolved by osteoclast cells. Then, the osteoblast cells work to form bone on the β-TCP particles (i.e., bone remodeling).

After implantation of ReBOSSIS^{®} at a bone defect site, biodegradable polymer (e.g., PLGA) in the electrospun fibers are gradually degraded such that the β-TCP particles buried in the fibers gradually become exposed, and the newly exposed β-TCP particles may recapture the BMP-2 (*i.e.* tBMP-2) that were adhered on the surface of the fibers. As the degradation of polymer proceeds, due to the recapture of BMP-2 (*i.e.* tBMP-2) by the newly exposed β-TCP particles, remodeling of bone continuously occurs throughout the network of the scaffold of biodegradable fibers, resulting in efficient bone formation at the bone defect site.

Due to the binding of BMP-2 (*i.e.* tBMP-2) to the β-TCP particles that are fixed to a surface of biodegradable fiber, the BMP-2 is prevented from leaking to outside of bone defect area. As a result, safety of using the BMP-2/ReBOSSIS^{®} is ensured.

### BRIEF DESCRIPTION OF DRAWINGS

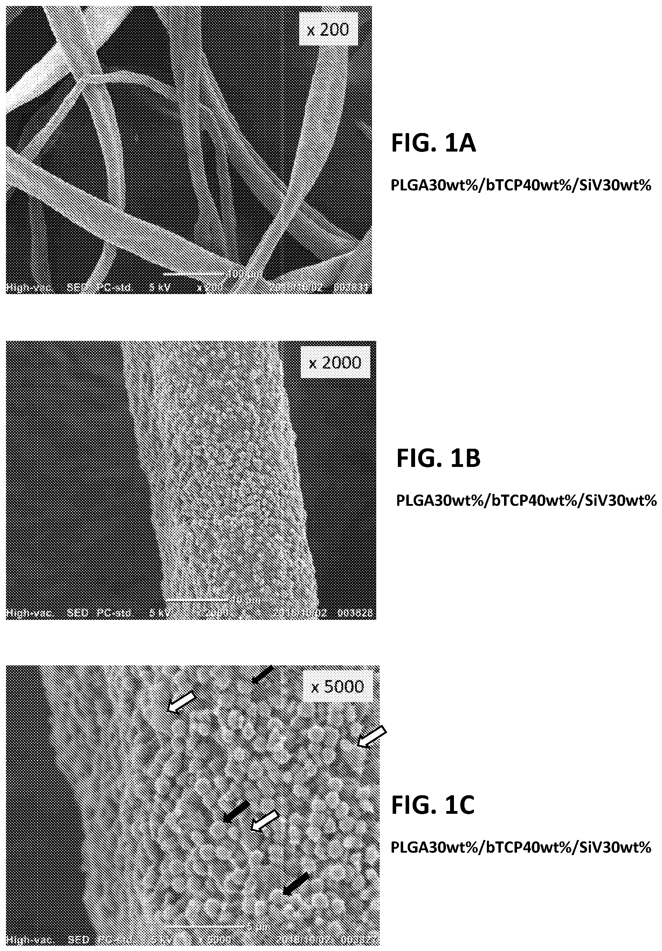

FIGs. 1A - 1F show electron microscope images of ReBOSSIS^{®} fibers. FIG. 1A show the image of several ReBOSSIS(85) fibers (PLGA 30 wt%, SiV 30 wt%, β-TCP 40 wt%) at 200x magnification, showing interstitial spaces between fibers in the cotton wool-like structure. FIG. 1B show the image of one ReBOSSIS(85) fiber at 2000x magnification. The calcium particles on the surface of the fiber are readily discernable. FIG. 1C shows the same fiber at 5000x magnification, in which the white arrows indicate the β-TCP particles and the dark arrows indicate the SiV particles.

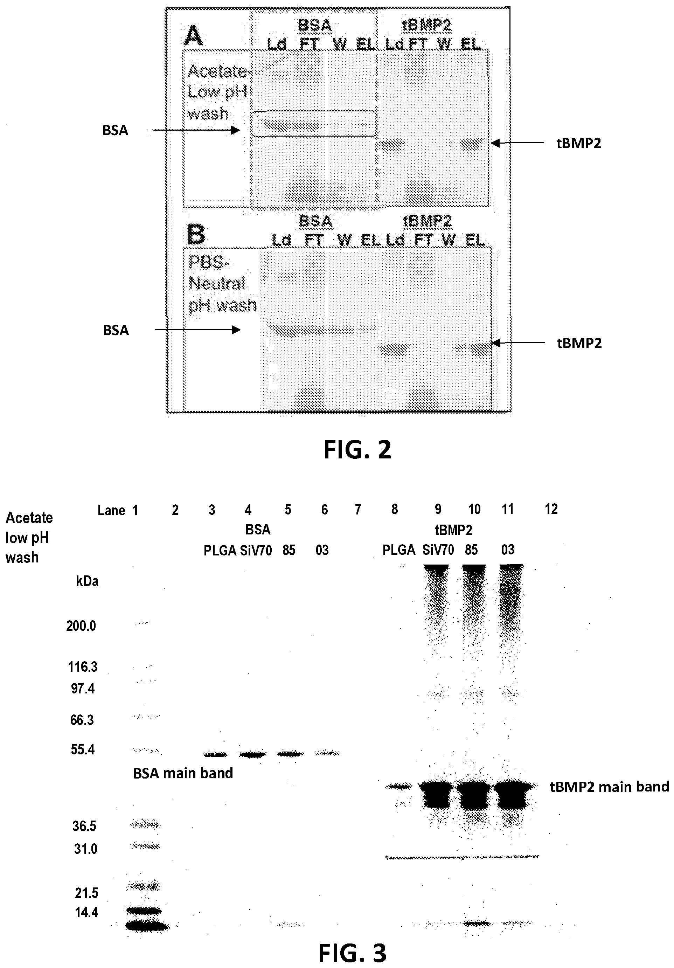

FIG. 2 shows an SDS-PAGE gel image illustrating the binding of tBMP-2 to ReBOSSIS^{®}. Panel A shows a gel image obtained using an acidic buffer (acetate buffer) for wash buffer, and Panel B shows a gel image obtained using a neutral buffer (PBS) for wash buffer. In each gel image, the four right lanes show results of analysis of tBMP-2, and the four left lanes show results of analysis of BSA.
FIG. 3 shows a gel image from the binding of tBMP-2 to several calcium containing materials. tBMP2 binds well to the materials (SiV70, ReBOSSIS(85), and ORB-03) containing β-TCP and/or SiV.

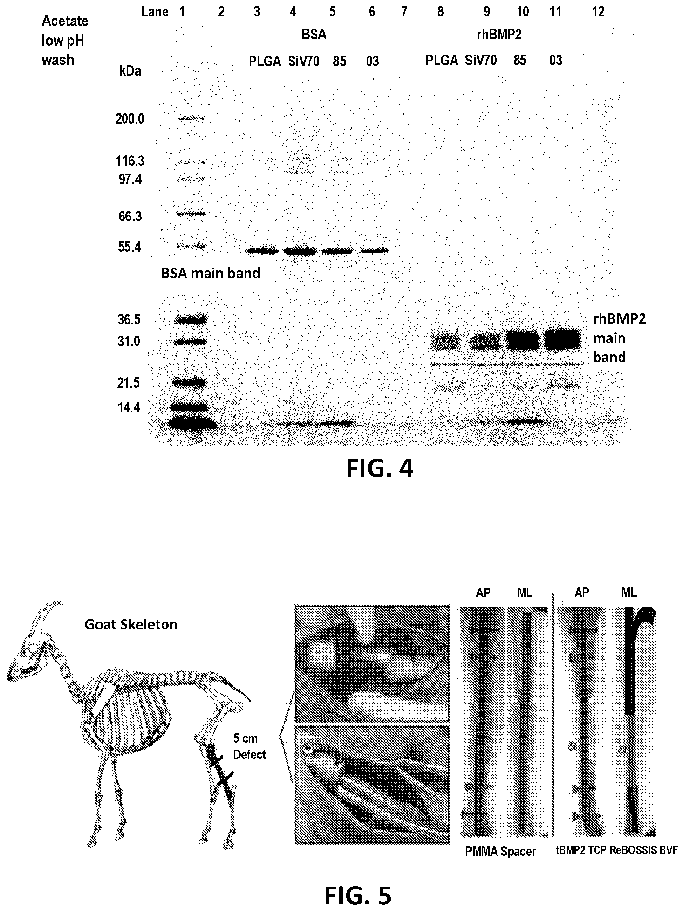

FIG. 4 shows a gel image from the binding of rhBMP-2 to several calcium containing materials. rhBMP-2 is only retained on materials containing β-TCP (ReBOSSIS (85) and ORB-03), but not on materials containing SiV.
FIG. 5 shows a schematic of Chronic Caprine Critical Defect (CCTD) Model. A 5-cm segment of critical defect is created in skeletally mature female goats during the pre-procedure. A 5-cm long x 2 cm diameter polymethylmethacrylate (PMMA) spacer is placed in the defect to induce a biological membrane. Four weeks later, the PMMA spacer is gently removed and replaced with the grafting materials. Orthogonal radiographs are taken every four weeks to assess defect healing. In the figure, AP represents craniocaudal, and ML represents mediolateral. White arrows indicate grafting material in placement of PMMA spacers.

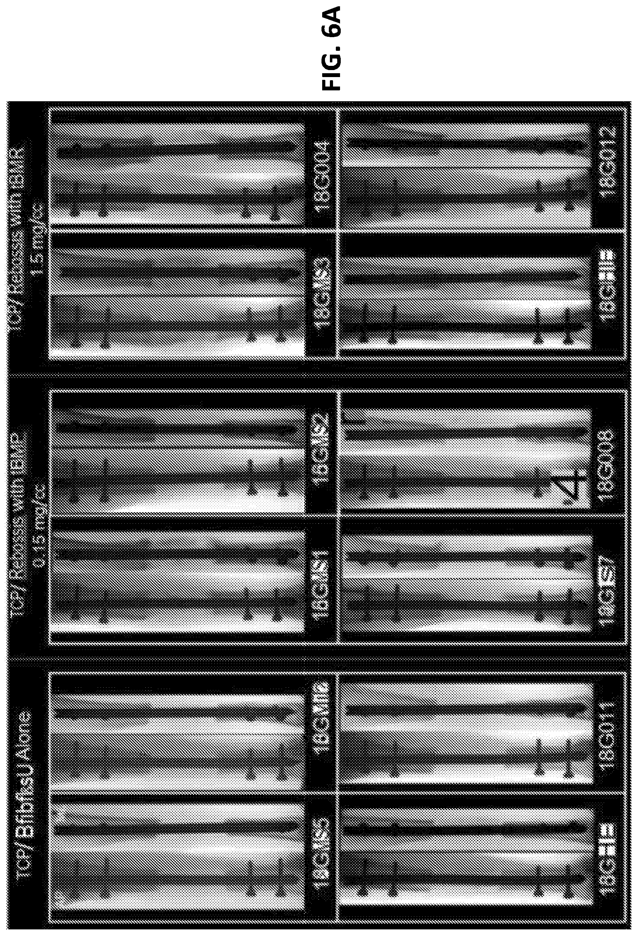

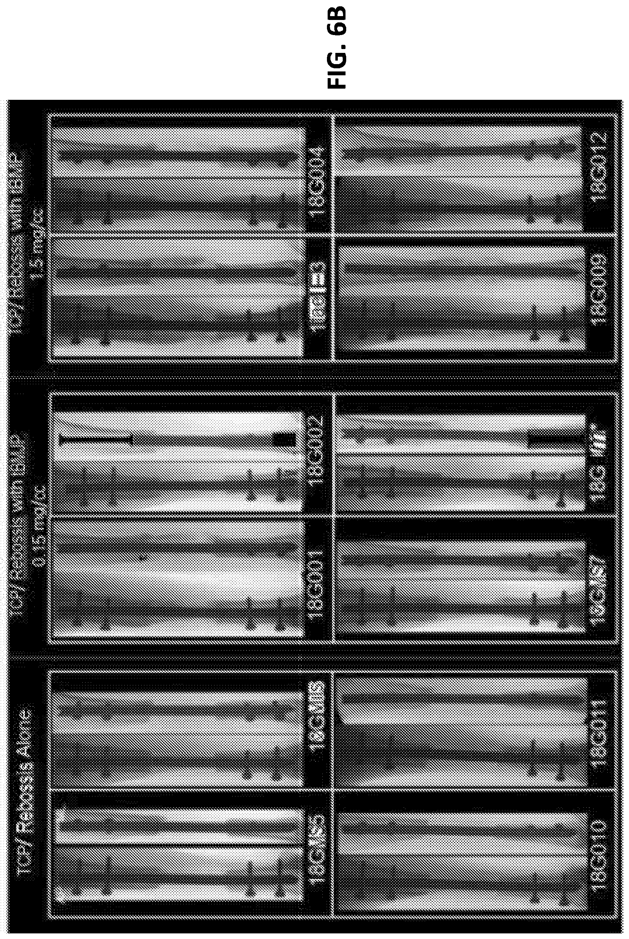

FIG. 6 shows the radiographs (mediolateral (ML) and craniocaudal (AP) projections) taken 8 weeks (A) and 12 weeks (B) after grafting surgery. 6 goats per treatment group. The tBMP-2-containing groups (Group 2 and 3) showed higher percentages of new bone formation compared to Group 1 (without tBMP-2).

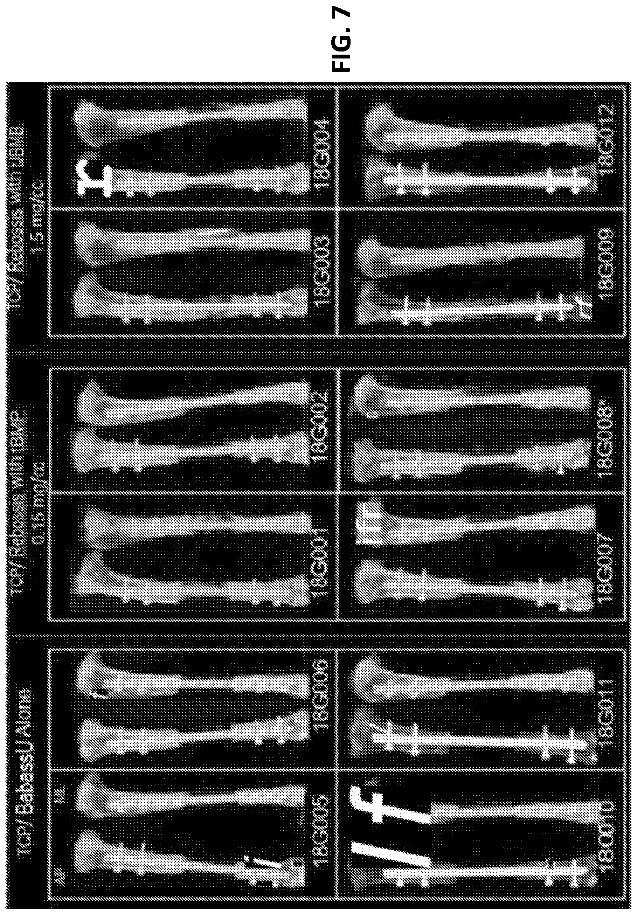

FIG. 7 shows radiographs (mediolateral (ML) and craniocaudal (AP) projections) of the 12 explanted tibias taken with a fixed x-ray machine. Large amount of new bone was obtained in the higher dose tBMP-2 group (1.5 mg/cc). The addition of tBMP-2 to TCP and ReBOSSIS^{®} enhanced the bone healing in the CCTD model.

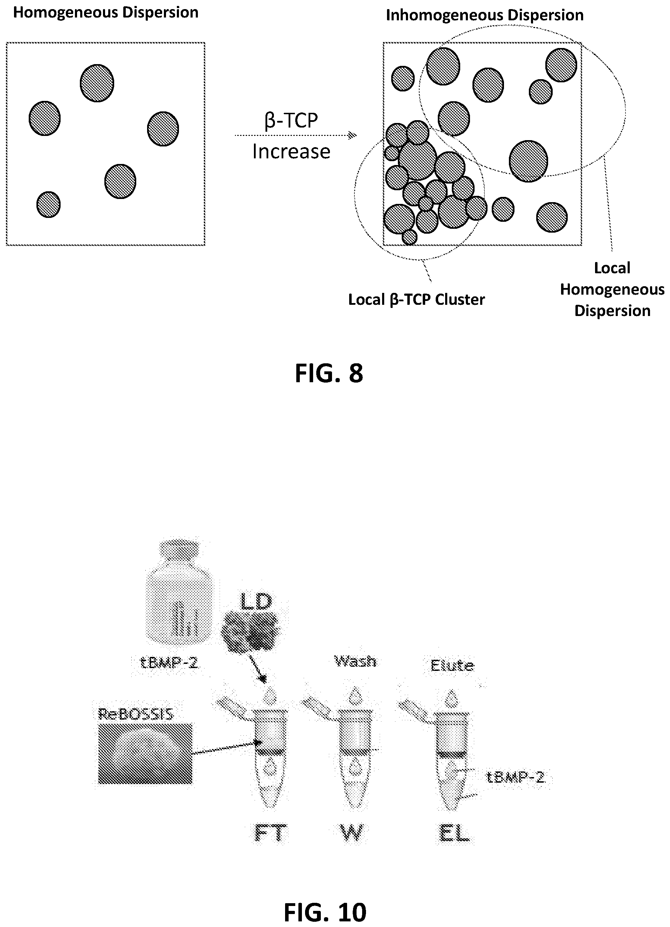

FIG. 8 is a conceptual diagram of percolation phenomenon, illustrating the formation of β-TCP particle clusters when the amounts of β-TCP particles exceeds the percolation threshold.

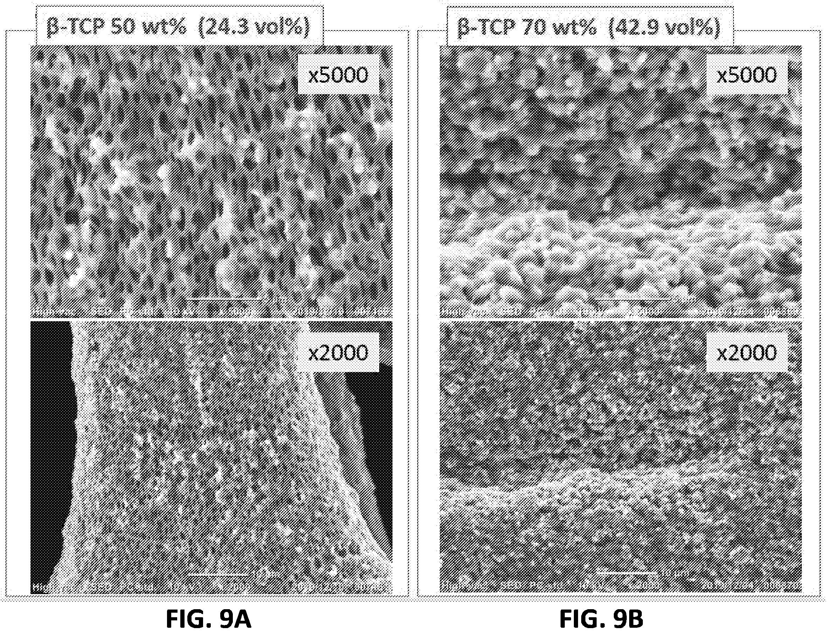

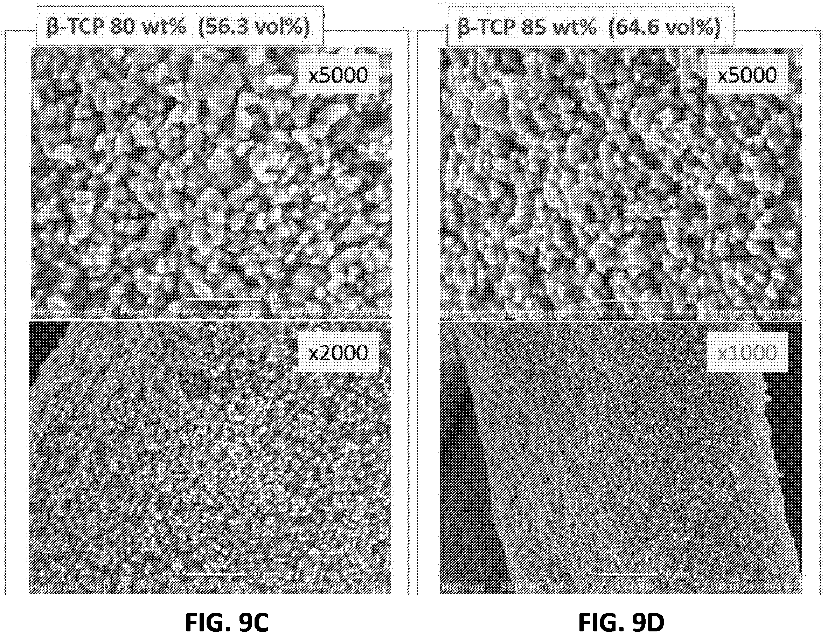

FIG. 9 (A) shows surface of electrospun PLGA fiber which contains β-TCP particles of 50 wt% (24.3 vol%). FIG. 9 (B) shows surface of electrospun PLGA fiber which contains β-TCP particles of 70wt% (42.9 vol%). FIG. 9 (C) shows surface of electrospun PLGA fiber which contains β-TCP particles of 80wt% (56.3 vol%). FIG. 9 (D) shows surface of electrospun PLGA fiber which contains β-TCP particles of 85wt% (64.6 vol%)
FIG. 10 is a diagram that explains the method of collecting samples for SDS-PAGE analysis.

### DETAILED DESCRIPTION

Embodiments of the invention relate to bone replacement materials that contain β-TCP and a bone morphogenetic protein-2 (i.e. tBMP-2). In addition, the bone replacement materials of the invention have a cotton wool-like structure such that the BMP-2, which is bound to a large surface area on the cotton wool-like structure, can interact with the biological fluids at the bone repair sites such that the osteoinduction process is facilitated.

Osteoinduction involves stimulation of osteoprogenitor cells to differentiate into osteoblasts that then begin new bone formation. In contrast, osteoconduction occurs when the bone graft material serves as a scaffold for new bone growth that is perpetuated by existing osteoblasts from the margin of the native bone surrounding the defect site.

The invention uses a targetable BMP-2. A targetable BMP-2 is a BMP protein fused with a β-TCP-binding peptide (i.e., a fusion protein) such that BMP-2 can bind tightly to β-TCP in the bone replacement materials. The β-TCP-binding peptide may be fused to the N- or C-terminus of the BMP-2.

BMP-2 have strong bone formation activities and are used in orthopedic applications, such as spinal fusion. However, BMP-2 may induce bone formation at the unintended sites, if they escape from the treatment sites. These BMP-2-associated complications occurred with relative high frequencies, ranging from 20% to 70% of cases, and these adverse effects could be potentially life threatening. (Aaron W. James et al., "A review of the Clinical Side Effects of Bone Morphogenetic Protein-2," Tissue Eng. Part B Rev., 2016, 22(4): 284-297). Thus, it is essential that one confine the BMPs at the treatment sites, e.g., by securely binding BMP-2 to the bone replacement/repair materials and not allow BMP-2 to diffuse away from the treatment sites.

Embodiments of the invention may use rhBMP-2 or BMP-2 fusion proteins that each contain one or more β-TCP binding peptides. These BMP-2 fusion proteins are referred to as targetable BMP-2 or tBMP-2. The tBMP-2 is designed to be used with β-TCP containing and/or SiV containing bone replacement/repair materials, in which the tBMP-2 bind tightly with β-TCP and/or SiV and would not diffuse away from the treatment sites, thereby eliminating or minimizing adverse effects.

The bone replacement/repair materials of the invention have a cotton wool-like structure made of biodegradable fibers that comprise β-TCP and a biodegradable polymer (e.g., poly(lactic-co-glycolic acid; PLGA). The tBMP-2 fusion proteins can bind tightly to β-TCP and/or SiV particles on these cotton wool-like structures and would not diffuse away from the treatment sites.

The cotton wool-like structure confers several advantages: (1) it contains a large interstitial space to allow biological fluids to readily permeate into the bone graft structure, (2) it offers a large surface area to allow ready release of calcium and phosphorus from β-TCP into the biological fluids; (3) it offers a large surface area to support/carry other bioactive or bone morphogenetic factors, such as rhBMP-2 or tBMP-2; and (4) it has a flexible structure that can be made to conform to the shape of the bone repair site.

The cotton wool-like structures are produced by electrospinning a solution containing a biodegradable polymer and β-TCP. Details of the formation of the cotton wool-like structures are described in U.S. patent Nos. 8,853,298, and 10,092,650, U.S. patent application publication Nos. 2016/0121024, and 2018/0280569. These cotton wool-like materials are available from Orthorebirth Co., Ltd. (Yokohama, Japan) under the tradename ReBOSSIS^{®}.

ReBOSSIS^{®} has a cotton-wool like structure formed of a plurality of electrospun biodegradable fibers containing β-TCP and optionally SiV particles, and a biodegradable polymer, such as poly(lactic-co-glycolic acid) (PLGA) or poly(lactic acid) (PLLA). The biodegradable fibers may contain β-TCP and optionally other calcium compound particles, such as silicon releasing calcium carbonate (vaterite) (SiV). Silicon-doped vaterite (SiV) particles have been found to have the ability to enhance cell activities in biodegradable composite materials. (Obata et al., "Enhanced in vitro cell activity on silicon-doped vaterite/poly(lactic acid) composites," Acta Biomater., 2009, 5(1): 57-62; doi: 10.1016/j.actbio.2008.08.004).

In ReBOSSIS^{®}, an electrospun biodegradable fiber contains a large amount of calcium compound particles (β-TCP and optionally SiV particles) distributed in the fiber. In typical ReBOSSIS^{®} fibers, the calcium compound particles (e.g., β-TCP particles or β-TCP + SiV particles) can account for about 30-85 wt%, preferably about 50-80 wt%, and more preferably about 70-80 wt%. If the amount of calcium compound particles exceeds 85 wt%, it becomes difficult to knead the mixture of PLGA and calcium compound particles to disperse the particles in the polymer.

The calcium compound particles are denser than the PLGA. For example, the PLGA has a density of 1.01 g/cm³, and β-TCP has a density of 3.14 g/cm³. Thus, the wt% and vol% may have a correlation as follows:

**Table 1. β-TCP content correlation**

| | | | | | | | | | |
|---|---|---|---|---|---|---|---|---|---|
| **wt%** | 90 | 80 | 70 | 60 | 50 | 40 | 30 | 20 | 10 |
| **vol%** | 74.3 | 56.3 | 42.9 | 32.5 | 24.3 | 17.7 | 12.1 | 7.4 | 3.5 |

In accordance with embodiments of the invention, the contents of the ReBOSSIS^{®} fibers may be referred to either in wt% or in vol%. For example, some ReBOSSIS^{®} fibers may contain β-TCP in an amount of about 25 - 65 vol% and the PLGA in an amount of about 75-35 vol%, more preferably β-TCP particles 40-60 vol% and PLGA 60-40 vol%.

In accordance with embodiments of the invention, the ReBOSSIS^{®} fibers have a portion of the calcium compound particles (e.g., β-TCP particles, or SiV particles, or β-TCP + SiV particles) exposed on the surface of the fibers, while the remaining portion of calcium compound particles are buried inside the fibers. For example, FIGs. 1A-1F show scanning electron micrographs of two ReBOSSIS^{®} samples: ReBOSSIS(85) comprises PLGA (30 wt% or 50.8 vol%), SiV (30 wt% or 27.4 vol%), and β-TCP (40 wt% or 21.8 vol%), and ORB-03 comprises PLGA (30 wt% or 57.1 vol%) and β-TCP (70 wt% or 42.9 vol%).

FIG. 1A shows the image of several ReBOSSIS(85) fibers (PLGA 30 wt%, SiV 30 wt%, β-TCP 40 wt%) at 200x magnification, showing interstitial spaces between fibers in the cotton wool-like structure. The large interstitial volume between the fibers facilitates the perfusion of biological fluids. FIG. 1B shows the image of one ReBOSSIS(85) fiber at 2000x magnification. The calcium particles on the surface of the fiber are readily discernable. FIG. 1C shows the same fiber at 5000x magnification, in which the white arrows indicate the β-TCP particles and the dark arrows indicate the SiV particles. The large number of calcium particles exposed on the surfaces of the fibers provide sites for binding by the BMP-2 or tBMP-2. In addition, the exposed calcium particles also facilitate interactions with osteoclasts and osteoblasts during remodeling and new bone formation.

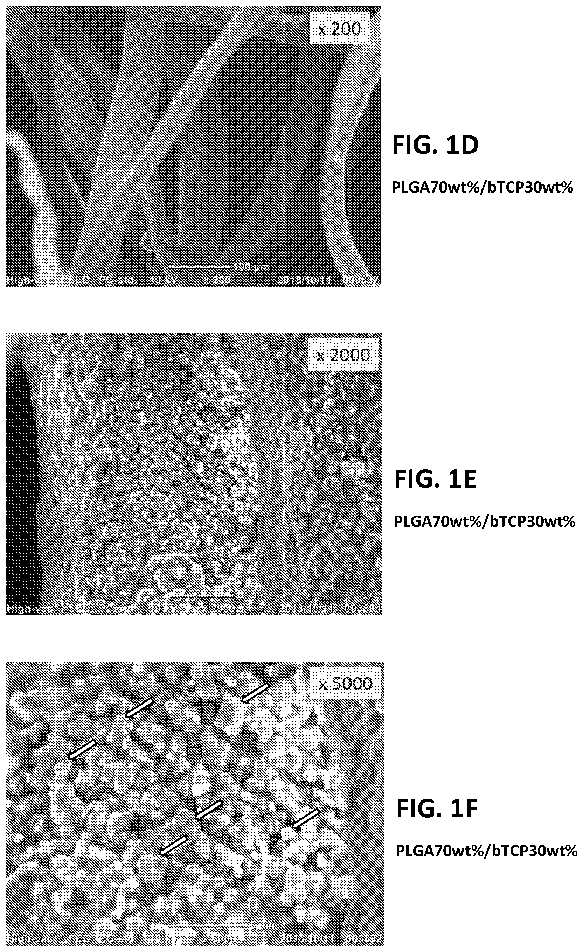

FIG. 1D shows the image of several ORB-03 fibers (PLGA 30 wt%/β-TCP 70 wt%) at 200x magnification, showing interstitial spaces between fibers in the cotton wool-like structure. The large interstitial volume between the fibers facilitates the perfusion of biological fluids. FIG. 1E shows the image of one ORB-03 fiber at 2000x magnification. The calcium particles on the surface of the fiber are readily discernable. FIG. 1F shows the same fiber at 5000x magnification, in which the white arrows indicate the β-TCP particles. The large number of calcium particles exposed on the surfaces of the fibers provide sites for binding by the BMP-2 or tBMP-2. In addition, the exposed calcium particles also facilitate interactions with osteoclasts and osteoblasts during remodeling and new bone formation.

In accordance with the invention, the fibers in ReBOSSIS^{®} preferably have diameters from about 5 µm to about 100 µm (including any number in the range), preferably from about 10 µm to about 100 µm, more preferably from about 10 µm to about 60 µm, such that calcium compound particles having a diameter of 1-5µm can be distributed in and on the fiber and the mechanical strength of the cotton wool-like structure is sufficient to maintain the desired shape after implantation of ReBOSSIS^{®} fibers at the site of a bone defect. The bulk density of a cotton wool-like structure of ReBOSSIS^{®} is about 0.01 to 0.2 g/cm3, preferably about 0.01 to 0.1 g/cm3, and the gaps between the fibers within the cotton wool-like structure are about 1-1000 µm, more preferably about 1-100 µm, such that body fluids can permeate throughout the gaps between the fibers and space for bone formation is ensured throughout the cotton wool-like structure.

After implantation of ReBOSSIS^{®} at a bone defect site, body fluids containing mesenchymal stem cells may come into contact with the BMP-2 (*i.e.,* tBMP-2) captured on the β-TCP particles. The BMP-2 then promotes osteoprogenitor cells to differentiate into osteoblasts. β-TCP particles that bind BMP-2 are gradually dissolved by osteoclasts or other biological active components. Then, the osteoblasts work to form new bone on the β-TCP particles as in a bone remodeling process.

After implantation of ReBOSSIS^{®} at a bone defect site, PLGA polymers in the electrospun fibers are gradually degraded such that β-TCP particles buried in the fibers would become exposed, and the newly exposed β-TCP particles would recapture the BMP-2 that were adhered on the surface of the fibers. As the degradation of PLGA proceeds, due to the recapture of BMP-2 by the newly exposed β-TCP particles, remodeling of bone continuously occurs throughout the network of the scaffold of biodegradable fibers, resulting in efficient bone formations at the bone defect sites.

In accordance with embodiments of the invention, the BMP-2 (e.g., rhBMP-2 or tBMP-2) binds to the β-TCP and/or SiV particles exposed on the surfaces of the fibers such that the BMP-2 is captured onto the ReBOSSIS^{®} fibers throughout the cotton-wool like structure. The fusion of the β-TCP-binding peptide may be to the N- or C-terminus of the BMP-2.

The tBMP-2 can be produced with conventional molecular biological techniques or other techniques known in the art (such as chemical or enzymatic couplings of the β-TCP-binding peptides to the BMPs). For example, the nucleic acid sequence for a β-TCP-binding peptide may be attached to the nucleic acid sequence of the BMP using polymerase chain reactions (PCR). Alternatively, the fusion protein nucleic acid construct may be chemically synthesized. The fusion protein construct is then placed into an appropriate expression vector at the restriction sites. The expression vector is then transfected into a protein expression system (e.g., E. coli, yeast cells, or CHO cells). The expressed proteins are then purified. To facilitate protein purification, a specific tag (e.g., a histidine tag) may be constructed into the expression construct. All these processes and techniques are conventional and routine. One skilled in the art would be perform these without undue experimentation.

In accordance with embodiments of the invention, a β-TCP binding peptide may comprise the amino acid sequence LLADTTHHRPWT (SEQ ID NO: 1), GQVLPTTTPSSP (SEQ ID NO: 2), VPQHPYPVPSHK (SEQ ID NO: 3), HNMAPATLHPLP (SEQ ID NO: 4), QSFASLTNPRVL (SEQ ID NO: 5), HTTPTTTYAAPP (SEQ ID NO: 6), QYGVVSHLTHTP (SEQ ID NO: 7), TMSNPITSLISV (SEQ ID NO: 8), IGRISTHAPLHP (SEQ ID NO: 9), MNDPSPWLRSPR (SEQ ID NO: 10), QSLGSMFQEGHR (SEQ ID NO: 11), KPLFTRYGDVAI (SEQ ID NO: 12), MPFGARILSLPN (SEQ ID NO: 13), QLQLSNSMSSLS (SEQ ID NO: 14), TMNMPAKIFAAM (SEQ ID NO: 15), EPTKEYTTSYHR (SEQ ID NO: 16), DLNELYLRSLRA (SEQ ID NO: 17), DYDSTHGAVFRL (SEQ ID NO: 18), SKHERYPQSPEM (SEQ ID NO: 19), HTHSSDGSLLGN (SEQ ID NO: 20), NYDSMSEPRSHG (SEQ ID NO: 21), or ANPIISVQTAMD (SEQ ID NO: 22), which are disclosed in U.S. Patent No. 10,329,327 B2.

In accordance with some embodiments of the invention, a β-TCP binding peptide may comprise two or more sequences selected from the above sequences. The two or more sequences may be directly connected to each other, or with a short peptide linker interspersed therebetween, to form a longer β-TCP binding peptide.

Due to the presence of β-TCP-binding peptides, the bindings of tBMP-2 to the β-TCP particles are very tight, thereby further preventing tBMP-2 from leaking to outside of bone defect areas. As a result, safety of using the tBMP-2/ReBOSSIS^{®} fibers is further ensured.
ReBOSSIS^{®}

ReBOSSIS^{®} is a bone-void-filling material having cotton wool-like structures formed of biodegradable fibers. Details of ReBOSSIS^{®} are explained in U.S. patent No. 8,853,298, U.S. patent No. 10,092,650, U.S. patent application publication No. 2016/0121024, and U.S. patent application publication No. 2018/0280569.

The diameters of the electrospun biodegradable fibers of ReBOSSIS^{®} range from about 5-100 µm, preferably about 10-100 µm, and more preferably about 10-60 µm. In contrast, the diameters of conventional electrospun fibers are usually several tens or several hundred nanometers (nm). Orthorebirth obtained thicker electrospun fibers by sending electrospinning (ES) solution to a large diameter nozzle at a fast rate and spinning the fibers by dropping the fiber from the top of ES apparatus to the bottom. The diameters of electrospun fibers become larger as the amounts of calcium compound particles are increased, eventually resulting in diameters of more than 60 µm. Because electrospinning is known for producing very thin nanofibers, the methods used by Orthorebirth for producing thicker fibers are unique. Details of the method of Orthorebirth is described in PCT/JP2019/036052 filed on September 13, 2019. By producing such thick electrospun biodegradable fibers, it becomes possible for the inventors to include a large amount of calcium compound particles in the fibers such that the particles are exposed on the fiber. Also, the thicker fibers have the mechanical strength to maintain the shape of the fibers after implantation at the bone repair sites.

Biodegradable fibers of ReBOSSIS^{®} contain large amounts of calcium particles (e.g., β-TCP, or β-TCP + SiV). Inclusion of such large amounts of calcium particles is achieved by using a kneading process. Briefly, a mixture for the biodegradable fiber and calcium particles is kneaded in a kneader with a strong force to produce a composite. The composite is then dissolved in a solvent (e.g., chloroform) to produce a spinning solution. Details of the kneading process is described in WO2017/188435 filed on April 28, 2017.

By adding calcium particles to the polymer in the kneader to knead the mixture of biodegradable polymer and calcium particles, calcium particles are homogeneously dispersed in the matrix polymer. However, if the volume ratio of calcium particles exceeds a threshold amount, due to the occurrence of percolation phenomenon, the particles can no longer maintain a homogeneous dispersion state, and cluster phase starts to appear (see FIG. 8). As a result of the cluster phase formation of the particles, some calcium particles are exposed on the surfaces of biodegradable fibers (see FIGs. 1A - 1F). This makes it possible for BMP-2 to bind to the β-TCP particles on the biodegradable fibers. According to experience of inventors, this percolation phenomenon starts to appear when the amounts of calcium particles exceed about 25 vol %.

FIGs. 9A -9D show EM images of ReBOSSIS^{®} fibers of the invention, illustrating the exposures of β-TCP particles on the biodegradable fiber increasing as the vol % ratio of β-TCP particles contained in the biodegradable fiber increases. FIG. 9A shows the fibers with β-TCP (50 wt%, 24.3 vol%); there are not many β-TCP particles exposed on the surface of the fiber. FIG. 9B shows the fibers with β-TCP (70 wt%, 42.9 vol%); there are many β-TCP particles exposed on the surface of the fiber. FIG. 9C shows the fibers with β-TCP (80 wt%, 56.3 vol%); there are many more β-TCP particles exposed on the surface of the fiber. FIG. 9D shows the fibers with β-TCP (85 wt%, 64.6 vol%); there are even more β-TCP particles exposed on the surface of the fiber.

In accordance with methods of the invention, a fiber spun from the nozzle of an electrospinning (ES) apparatus is projected into a collector filled with ethanol, in which the fiber is collected in a form of cotton wool-like structure. In the collector, the solvent (e.g., chloroform) in the fibers is removed by dissolution in ethanol.

The cotton wool-like structures provide a large surface area for BMP-2 binding and sufficient interstitial spaces for biological fluids that contain cells that can participate in bone formation to infuse/permeate, thereby enhancing the osteoinduction processes.
tBMP-2

The invention uses targetable BMP-2 (tBMP-2), in which β-TCP binding peptides are fused with BMP2. tBMP-2 is fused with a β-TCP binding peptide developed by one of the inventors of this invention. Details of the β-TCP binding peptides are explained in U.S. patent publication No. 10,329,327 B2 and in *"*Tethering of Epidermal Growth Factor (EGF) to Beta Tricalcium Phosphate (β TCP) via Fusion to a High Affinity, Multimeric β-TCP binding Peptide: Effects on Human Multipotent Stromal Cells/Connective Tissue Progenitors," Alvarez et al., PLoS ONE DOI: 10.1371/journal.pone.0129600, June 29, 2015.

In accordance with embodiments of the invention, a β-TCP binding peptide may comprise the amino acid sequence LLADTTHHRPWT (SEQ ID NO: 1), GQVLPTTTPSSP (SEQ ID NO: 2), VPQHPYPVPSHK (SEQ ID NO: 3), HNMAPATLHPLP (SEQ ID NO: 4), QSFASLTNPRVL (SEQ ID NO: 5), HTTPTTTYAAPP (SEQ ID NO: 6), QYGVVSHLTHTP (SEQ ID NO: 7), TMSNPITSLISV (SEQ ID NO: 8), IGRISTHAPLHP (SEQ ID NO: 9), MNDPSPWLRSPR (SEQ ID NO: 10), QSLGSMFQEGHR (SEQ ID NO: 11), KPLFTRYGDVAI (SEQ ID NO: 12), MPFGARILSLPN (SEQ ID NO: 13), QLQLSNSMSSLS (SEQ ID NO: 14), TMNMPAKIFAAM (SEQ ID NO: 15), EPTKEYTTSYHR (SEQ ID NO: 16), DLNELYLRSLRA (SEQ ID NO: 17), DYDSTHGAVFRL (SEQ ID NO: 18), SKHERYPQSPEM (SEQ ID NO: 19), HTHSSDGSLLGN (SEQ ID NO: 20), NYDSMSEPRSHG (SEQ ID NO: 21), or ANPIISVQTAMD (SEQ ID NO: 22). In accordance with some embodiments of the invention, a β-TCP binding peptide may comprise two or more sequences selected from the above sequences. The two or more sequences may be directly connected to each other, or with a short peptide interspersed therebetween, to form a longer β-TCP binding peptide.
Binding BMP-2 to ReBOSSIS^{®}

In accordance with embodiments of the invention, BMP-2 can bind to the ReBOSSIS^{®} fibers. To evaluate the properties of BMP-2 binding to ReBOSSIS^{®} fibers, the following experiment was performed (using tBMP-2 or rhBMP-2 as an example). In this experiment, biodegradable fibers of ReBOSSIS^{®} contains PLGA (30 wt%) and β-TCP particles (40 wt%) and SiV (silicon doped calcium carbonate of vaterite phase) particles (30 wt%). The β-TCP particles and SiV particles are distributed in and on the fibers. A portion of the particles is exposed outside on the surfaces of the fibers macroscopically.

Four sample solutions were prepared. The concentrations of tBMP-2 and rhBMP-2 in the following four sample solutions were compared with a control sample using Poly-Acrylamide Gel Electrophoresis (SDS-PAGE). Bovine serum albumin (BSA) was used as the control sample.

Specifically, the following reagents were prepared: (a) tBMP-2 (Theradaptive TCP binding BMP-2, in 10 mM Na-Acetate, 0.1M NaCl with or without 0.1 M Urea, pH 4.75); (b) BSA Stock solution: 42 mg/ml dissolved in Acetate Wash Buffer (store at -20 °C); (c) Acetate Wash Buffer: 5m M Na-acetate pH 4.75, 100 mM NaCl; (d) ReBOSSIS^{®} (OrthoRebirth); and (e) PBS (Roche, cat# 11666789001, 1X solution = 137 mM NaCl, 2.7 mM KCl, 10 mM Na₂HPO₄, 1.8 mM KH₂PO₄, pH 7.0).

Method: Bind 20 µg tBMP-2 or BSA / mg ReBOSSIS^{®} (total 200 µg tBMP-2 and 100 mg ReBOSSIS^{®}), wash, elute, and load onto Non-reducing SDS-PAGE. Specific protocols are as follows:

### Preparation

1. Weigh 10 mg ReBOSSIS^{®} in spin tubes;
2. Prepare BSA: Take 29 µl BSA Stock and add 971 µl of acetate buffer to achieve a diluted BSA solution, 1.22 mg/ml at pH 4.75.
3. Prewash ReBOSSIS^{®} in acetate wash buffer by adding 500 µl acetate wash buffer in each tube. Mix well end over end, let it incubate for 5 minutes. Spin down in a microfuge. To remove the buffer, place tip at bottom of tube and pipet. ReBOSSIS^{®} will remain in the tube.
4. Gel Sample: Set aside some BSA Load and tBMP2 load for later SDS-PAGE analysis.

### Assay

5. Add 200 µg tBMP2 to a 10 mg prewashed ReBOSSIS^{®} in a total volume of 164 µl;
6. Add 200 µg BSA to a 10 mg prewashed ReBOSSIS^{®} in a total volume of 164 µl

### Example Setup

| **Tube No.** | **BSA Control** | **tBMP-2** |
|---|---|---|
| BSA (1.22 mg/ml) | 164 | |
| tBMP2 (2.41 mg/ml) | - | 83 |
| Acetate pH 4.75 (100 mM NaCl) | - | 81 |

7. Bind for 30 minutes (binding is nearly complete at 20 minutes);

### Collect Unbound Material

8. Spin tubes for 4-5 minutes at the highest speed in a microfuge.
9. Insert a pipet into the supernatant at the top of the tube and pipet out the non-bound into a tube. (Note: Make sure not to get ReBOSSIS^{®} in your pipet tip. We take out excess amounts to run our gel. Then we place a tip in the bottom of the tube and discard the remainder);

### Wash

10. Add 500 µl of either PBS or acetate wash buffer;
11. Gently Vortex. Mix end over end for 5 minutes, then gently vortex again;
12. Extract Wash by spinning tubes for 4-5 minutes at the highest speed in a microfuge. Keep the wash;

### Elute

13. Elute by adding 164 µl non-reducing 1X SDS PAGE gel dye (Without β-mercaptoethanol, as that will destroy the BMP2 dimer);
14. Vortex gently, incubate 5 minutes, repeat vortex;
15. Collect the eluted material by spinning tubes for 4-5 minutes at the highest speed in a microfuge;
16. Load gel as follows: ReBOSSIS^{®} Load:10 µl, Non-Bound 10 µl, Wash 10µl, Elution 11 µl.

The samples for SDS PAGE analysis are labeled as follows (as shown in FIG 10):
Ld: Loading sample solution containing a known amount of tBMP-2 or BSA.
FT: Flow-through sample solution obtained by collecting the fraction that flowed through ReBOSSIS^{®} after Ld was provided to ReBOSSIS^{®}.
W: Wash sample solution obtained by collecting the fraction that went through the protein-containing ReBOSSIS^{®} after a wash buffer was provided.
   - If protein that was bound to ReBOSSIS^{®} became separated by wash buffer, the fraction that flowed out after washing would contain the separated protein.
   - Assuming that the pH condition of the implant site is either acidic or neutral, two types of wash buffers (PBS pH 7.0 and acetate buffer pH 4.5) were prepared and used to conduct the experiments.
EL: Elution sample solution obtained by collecting the fraction after an elution buffer was provided to the ReBOSSIS^{®} after being washed by a wash buffer.

### Evaluation of the binding of protein (SDS-PAGE)

The binding of tBMP-2 (or BSA) to ReBOSSIS^{®} was evaluated on SDS-PAGE, and a staining solution was used to detect the protein bands. The detected protein appears as a band in a lane. By comparing the signal intensity of an electrophoresis band of a sample having a known amount of protein with the signal intensity of an electrophoresis band of a sample with an unknown amount of protein, the unknown amount of protein can be quantitatively estimated. By using software for image analysis, the intensities of the signals can be quantitatively analyzed.

In this experiment, the gel image was compared by eye observations. A blue band shown in a lane was produced by staining the protein (tBMP2 or BSA) with a blue dye. A denser band indicates a larger amount of the protein.

SDS-PAGE was conducted at a constant voltage of 130V. NuPAGE 4-12% Bis-Tris Protein Gels, 1.0 mm, 12-well (Life Technologies, cat#NP0322BOX) was used, and NuPAGE MOPS SDS Running Buffer (20X) (Life Technologies cat#NP0001) was used as the electrophoresis buffer. In order to stain the protein after electrophoresis is completed, Gelcode Blue Sage Protein Stain (Thermos Scientific, cat # 24596) was used.

As shown in FIG. 2, Panel A shows a gel image obtained using an acidic buffer (acetate buffer) for wash buffer, and Panel B shows a gel image obtained using a neutral buffer (PBS) for wash buffer. In each gel image, the four right lanes show results of analysis of tBMP2, and the four left lanes show results of analysis of BSA.

In the Ld lanes (loading samples), the positions of main bands of tBMP-2 and BSA can be identified. If tBMP-2 or BSA is contained in the FT, W, or EL lane, its band is expected to appear at the same position as that in the Ld lane.

In FIG. 2, the area surrounded by dotted lines shows gel image of the sample that was prepared using BSA under condition A. The position of the main band of BSA is indicated by a rectangular solid line box, which is denoted as BSA main band. From a comparison of the intensities of the main bands of BSA in each lane, it is clear that the FT and Ld lanes show similar levels of proteins, indicating that most BSA does not bind ReBOSSIS^{®} and flow right through. That is, BSA acts as a negative control that does not bind ReBOSSIS^{®} fibers. Although the W and EL lanes also show trace amount of BSA bands, the levels of BSA bands in the W and EL lanes are at much lower levels, as compared with that of the Ld lane, indicating that little BSA was bound to ReBOSSIS^{®}.

In the neutral pH buffer (panel B), BSA show non-specific bindings to the ReBOSSIS^{®} fibers. Most BSA came through in the flow through fraction (FL), indicating that most BSA does not bind the ReBOSSIS^{®} fibers. In the wash fraction (W), some BSA continues to come through, but the amount is less than the flow through faction. The amount is even less in the elution (EL) fraction. These results indicate non-specific sticking of BSA to the ReBOSSIS^{®} fibers.

In contrast, tBMP-2 binds well to ReBOSSIS^{®} and very little came out in the flow through (FT) or the wash (W) fractions, either using an acidic buffer or a neutral pH buffer. The bound tBMP-2 came out only after elution (EL). (FIG. 2, Panel A and Panel B), indicating specific binding of tBMP-2 to the ReBOSSIS^{®} fibers.

From this experiment, it was confirmed that tBMP-2 can bind tightly to ReBOSSIS^{®} Fibers, and that tBMP-2 bound to ReBOSSIS^{®} fibers is not separated from ReBOSSIS^{®} fibers by acidic or neutral wash buffer.

From this experiment, it was confirmed that under both neutral and acidic conditions, greater than 98% of tBMP-2 was bound and retained on ReBOSSIS^{®}. This means that even under the effect of osteoclast resorption, binding of tBMP-2 to ReBOSSIS^{®} continues.

### Comparison of retention between tBMP-2 and rhBMP-2 (Infuse)

This experiment is to compare the binding properties of tBMP-2 and rhBMP-2 (INFUSE) to ReBOSSIS^{®} fibers. The tests were conducted at several composition ratios as shown in the following table:

| **No.** | **Sample Name** | **Composite Material** | **PLGA** | **SiV** | **β-TCP** |
|---|---|---|---|---|---|
| 1 | PLGA100 | Negative control | 100 wt% | - | - |
| 2 | SiV70 | SiV | 30 wt% | 70 wt% | - |
| 3 | ReBOSSIS (85) | SiV and β-TCP | 30 wt% | 30 wt% | 40 wt% |
| 4 | ORB-03 | β-TCP | 30 wt% | - | 70 wt% |

| | | | | | |
|---|---|---|---|---|---|
| PLGA: poly(lactic-co-glycolic acid) SiV: Siloxane-containing vaterite (a form of calcium carbonate, CaCO3) β-TCP: β-Tricalcium phosphate | | | | | |

The binding protocols are as described above. FIG. 3 shows the results of bindings of tBMP-2 to the various materials. On the materials (SiV70, ReBOSSIS (85), and ORB-03) containing β-TCP and/or SiV (siloxane-containing vaterite), tBMP-2 is well retained. The retention of tBMP-2 is clearly different from that of BSA.

FIG. 4 shows results for recombinant human BMP-2 (rhBMP-2). rhBMP-2 is only retained on materials containing β-TCP (ReBOSSIS (85) and ORB-03), but not on material containing SiV. The binding of tBMP-2 is stronger than that of rhBMP-2. Nevertheless, rhBMP2 is still good to use on ReBOSSIS^{®}. Because retention of rhBMP-2 by ReBOSSIS^{®} is less than that of tBMP-2, one can predict that tBMP-2 is less likely to leak out of the treatment site. Thus, preferred embodiments of the invention may use tBMP-2, which would have fewer (if any) adverse effects, as compared with rhBMP-2 (e.g., INFUSE^{®} Bone Graft).

### Evaluation of ReBOSSIS/tBMP2 in a Chronic Caprine Tibial Defect (CCTD) Model

To evaluate the utility of tBMP-2/ReBOSSIS^{®} in bone repair, the efficacy of targetable BMP-2 (tBMP-2) on ReBOSSIS^{®} was evaluated in the CCTD model, which is a challenging long-bone segmental defect goat model. It is expected that prolonged local retention of surface-bound tBMP-2 at implantation sites improves the safety and efficacy of orthopedic procedures to correct long-bone segmental defects compared to the current practice.

### Study Design

### Animal Selection

Twelve (12) female Spanish Boer goats weighing between 40 - 60 kg were used for the study. They were divided into the following three experimental groups:
- Group 1 - TCP + ReBOSSIS^{®} + bone marrow aspirate (BMA) alone
- Group 2 - TCP + ReBOSSIS^{®} + BMA + tBMP-2 @0.15 mg/cc defect
- Group 3 - TCP + ReBOSSIS^{®} + BMA + tBMP-2 @1.5 mg/cc defect
TCP, tricalcium phosphate granules; BMA, bone marrow aspirate

### CCTD Model

The CCTD was conceived and developed by Drs. Muschler (Cleveland Clinic), Pluhar (University of Minnesota), Bechtold (University of Minnesota), and Wenke (ISR). The CCTD model is intended to "raise the bar" for large animal models and better match the challenging clinical biological settings where current treatments for large bone defects continue to fail with unacceptable frequency.

The CCTD model involves a critical size (5 cm) segmental tibial defect of bone. Several features distinguish the CCTD model from acute defect models:
1. 2 cm of periosteum is removed from each end of the defect site, creating a 9 cm segment of periosteum (the 5-cm defect + 2 cm on either side),
2. 10 grams of skeletal muscle around the defect site,
3. the intramedullary canal is reamed removing marrow and endosteal bone adjacent to the defect site, and
4. a PMMA spacer is placed in the defect for 4 weeks prior to grafting. This allows the spacer to be enveloped by a fibrous "induced membrane" (IM) or "Masquelet membrane."
5. Each animal undergoes two surgeries defined here as the "Pre-procedure" to create these biological conditions and the "Treatment Procedure," in which clinically relevant treatment scenarios can be implemented.

FIG. 5 shows a Schematic of Chronic Caprine Critical Tibial Defect (CCTD) Model. A 5-cm segment of critical defect is created in skeletally mature female goats during the pre-procedure. A 5-cm long x 2 cm diameter polymethylmethacrylate (PMMA) spacer is placed in the defect to induce a biological membrane. Four weeks later, the PMMA spacer is gently removed and replaced with the grafting materials. Orthogonal radiographs are taken every four weeks to assess defect healing. In the figure, AP represents craniocaudal, and ML represents mediolateral. White arrows indicate grafting material in placement of PMMA spacers.

The Pre-Procedure comprises the following essential features:
1. Creation of a craniomedial skin incision and excision of a 5-cm segment of tibial diaphysis and periosteum.
2. Excision of an additional 2 cm of periosteum on the proximal and distal bone segments.
3. Debridement of 10 cm³ of tibialis anterior and gastrocnemius muscles.
4. Placement of interlocking intramedullary nail with custom spacer clamp to maintain 5 cm defect.
5. Placement of a pre-molded 5 cm long x 2 cm diameter PMMA spacer around the nail in the defect.
6. Irrigation of the wound normal (0.9 %) saline and wound closure.

The Treatment Procedure to be performed 4 weeks after the Pre-Procedure comprises:
1. Opening the previous skin incision on the craniomedial aspect of the tibia.
2. Opening the "induced membrane" around the PMMA spacer using a "bomb bay door opening."
3. Spacer removal without damaging the membrane or nail.
4. Collection of appropriate tissue samples as defined below.
5. Placement of appropriate treatment into the defect.
6. Closure of the induced membrane with 3-0 nylon to provide an intrinsic marker and wound closure.

### Radiographic Analysis:

Fluoroscopic imaging of the tibiae, anteroposterior (AP) and mediolateral (ML) projections were performed after the spacer procedure (week 0), the graft procedure (week 4), and follow-ups (week 8 and week 12). Radiographs were obtained after euthanasia (after soft tissue were dissected) 12 weeks after the grafting procedure.

### Sample Preparation

Sample composition: 5 cc TCP + 50 cc ReBOSSIS^{®} + 6 cc BMA (with or without tBMP-2).

### Binding tBMP2 to ReBOSSIS^{®}

1. In a sterile environment, tease out 50cc ReBOSSIS^{®} in a petri-dish making sure it is spread out in one uniform layer;
2. Add 30mL binding buffer to ReBOSSIS^{®}, by gently pipetting over the exposed surface and submerge ReBOSSIS^{®} in the solution, bind for 20 min;
3. Take out 30mL binding buffer using a 10mL pipette, by holding it vertically and pushing the tip to the surface of the place. While holding it to the surface suck up liquid carefully. Move the pipette to other areas to make sure to collect as much liquid as possible. Monitor the recovery volume;
4. Add 40mL sterile PBS onto ReBOSSIS^{®} to wash for 10 min. Add the same way as described in step 2;
5. Take out the 40mL PBS using a 10mL pipette as described in step 3, store PBS in a 50mL conical tube;
6. Repeat 1x step 4-5;
7. Put the lid on the Petri dish and Parafilm the edge to keep the tBMP2/ReBOSSIS^{®} sealed.

### Binding tBMP2 to TCP

1. Measure the desired amount of TCP and place into a sterile tube.
2. Sterilize TCP by filling the tube with 70% Ethanol and incubating for 2-4 hours or overnight.
3. Wash TCP with sterile double deionized Water 3x to remove alcohol.
4. Wash TCP for 5 minutes in TCP binding buffer (10mM Sodium Acetate pH 4.75, 100mM NaCl) while gently agitating.
5. Wash TCP with sterile PBS to remove the TCP binding buffer.
6. Add the appropriate amount of tBMP2 to the TCP in the tube.
7. Add TCP binding buffer sufficient to cover the TCP.
8. Mix gently for 2 hours.
9. Wash with PBS X2 to remove the TCP binding buffer.
10. Store tBMP-2/TCP in the sterile container at 4°C.

### Time of Surgery

1. Open one dish containing tBMP2/ReBOSSIS^{®}.
2. Find a corner of the same dish, decant the 5 ccs of tBMP-2 coated TCP. Then apply the 6 cc of bone marrow aspirate to the TCP and distribute evenly.
3. Use a sterile spatula transfer tBMP-2/TCP/BMA on top of the ReBOSSIS^{®}, making sure it spreads out evenly across the entire pile of ReBOSSIS^{®}.
4. Use sterile gloved hand gently pad the abovementioned tBMP-2/TCP/BMA so it's evenly distributed on the ReBOSSIS^{®} like a layer of fine pebbles.
5. Gently roll ReBOSSIS^{®} up like a burrito and mix and shape as needed

### Results

The surgical handling property of grafting materials was greatly enhanced by the addition of ReBOSSIS^{®}. The tBMP-2-containing groups (Group 2 and 3) showed higher percentages of new bone formation compared to Group 1. FIG. 6A shows the radiographs (mediolateral (ML) and craniocaudal (AP) projections) taken 8 weeks after grafting surgery, and FIG. 6B shows the radiographs (mediolateral (ML) and craniocaudal (AP) projections) taken 12 weeks after grafting surgery. Six (6) goats were used per treatment group.

The post-explant x-rays showed that no new bone was obtained in any of the defect sites for all 4 goats in Group 1 (TCP + ReBOSSIS^{®} + BMA). In Group 2 (low dose tBMP2), one of 4 goats had about 75 % of new bone growth filled in the defect site, the other 3 goats had less than 25 % new bone filled in the defect sites. In Group 3 (higher dose tBMP-2), 2 goats presented bone union and 2 goats presented less than 50% new bone. These data indicate that the tBMP2 addition to the scaffold did increase new bone formation.

FIG. 7 shows radiographs (mediolateral (ML) and craniocaudal (AP) projections) of the 12 explanted tibias taken with a fixed x-ray machine. Large amount of new bone was obtained in the higher dose tBMP-2 group (1.5 mg/cc).

As shown in these results, ReBOSSIS^{®} greatly enhanced the surgical handling property of the implant material. The addition of tBMP2 to TCP and ReBOSSIS^{®} enhanced the bone healing in the CCTD model. These results indicate that embodiments of the invention would be superior than presently used materials for bone repair. While these particular examples use tBMP-2, rhBMP-2 would produce the same results as having been demonstrated before.

While embodiments of the invention have been illustrated with a limited number of examples, the scope of the protection should only be limited with the attached claims.

## Claims

1. A bone regeneration material comprising a structure of biodegradable fibers comprising beta-tricalcium phosphate (b-TCP) and a biodegradable polymer, and
a bone morphogenetic protein-2 (BMP-2),
wherein the BMP-2 is bound to the b-TCP, wherein the BMP-2 is a targetable BMP-2 protein fused with a b-TCP-binding peptide, and the BMP-2 is bound to the b-TCP via the b-TCP-binding peptide, wherein the b-TCP is 30-85 weight % of the structure, and
wherein the biodegradable fibers comprise fibers with a thickness of about 5 micrometers to about 100 micrometers, and the biodegradable fibers are formed by electrospinning.

2. The bone regeneration material of claim 1, wherein the biodegradable polymer comprises polylactic co-glycolic acid) (PLGA).

3. The bone regeneration material of claim 1 or claim 2, wherein the structure comprises a large interstitial space to allow biological fluids to readily permeate into the structure.

4. The bone regeneration material of any preceding claim, wherein the structure comprises a large surface area to allow release of calcium and phosphorus from b-TCP into biological fluids.

5. The bone regeneration material of any preceding claim, wherein the structure comprises a large surface area to support binding of BMP-2 to the structure.

6. The bone regeneration material of any preceding claim, wherein the structure is flexible and can be made to conform to the shape of a bone repair site.

7. The bone regeneration material of any preceding claim, wherein the b-TCP is distributed in the fibers.

8. The bone regeneration material of any preceding claim, wherein the b-TCP is exposed on the surface of the fibers and buried inside the fibers.

9. The bone regeneration material of claim 8, wherein the exposed b-TCP provide sites for binding to the BMP-2.

10. The bone regeneration material of claim 8 or claim 9, wherein the exposed b-TCP facilitates interaction with osteoclasts and osteoblasts during remodeling and new bone formation.

11. The bone regeneration material of any preceding claim, wherein gaps between the biodegradable fibers are 1 to 1000 micrometers, and allow for body fluid to permeate through the gaps.

12. The bone regeneration material of any one of claims 1 to 11, wherein the biodegradable fibers have the mechanical strength to maintain the shape of the fibers after implantation at the bone repair site.

13. The bone regeneration material of any one of claims 1 to 12, for use in bone repair.

## Patentansprüche

1. Knochenregenerationsmaterial, das eine Struktur aus biologisch abbaubaren Fasern umfasst, die Beta-Tricalciumphosphat (b-TCP) und ein biologisch abbaubares Polymer umfasst, und
ein knochenmorphogenetisches Protein-2 (BMP-2),
wobei das BMP-2 an das b-TCP gebunden ist, wobei das BMP-2 ein targetierbares BMP-2-Protein fusioniert mit einem b-TCP-Bindungspeptid ist und das BMP-2 an das b-TCP über das b-TCP-Bindungspeptid gebunden ist, wobei das b-TCP 30-85 Gewichts-% der Struktur ist, und
wobei die biologisch abbaubaren Fasern Fasern mit einer Dicke von etwa 5 Mikrometern bis etwa 100 Mikrometern umfassen und die biologisch abbaubaren Fasern durch Elektrospinnen gebildet sind.

2. Knochenregenerationsmaterial nach Anspruch 1, wobei das biologisch abbaubare Polymer Polymilchsäure-Co-Glykolsäure) (PLGA) umfasst.

3. Knochenregenerationsmaterial nach Anspruch 1 oder Anspruch 2, wobei die Struktur einen großen interstitiellen Raum umfasst, um zu ermöglichen, dass biologische Fluide leicht in die Struktur dringen.

4. Knochenregenerationsmaterial nach einem vorhergehenden Anspruch, wobei die Struktur einen großen Oberflächenbereich umfasst, um Freisetzung von Calcium und Phosphor aus b-TCP in biologische Fluide zu ermöglichen.

5. Knochenregenerationsmaterial nach einem vorhergehenden Anspruch, wobei die Struktur einen großen Oberflächenbereich umfasst, um Bindung von BMP-2 an die Struktur zu unterstützen.

6. Knochenregenerationsmaterial nach einem vorhergehenden Anspruch, wobei die Struktur flexibel ist und hergestellt werden kann, um sich an die Form einer Knochenreparaturstelle anzupassen.

7. Knochenregenerationsmaterial nach einem vorhergehenden Anspruch, wobei das b-TCP in den Fasern verteilt ist.

8. Knochenregenerationsmaterial nach einem vorhergehenden Anspruch, wobei das b-TCP auf der Oberfläche der Fasern freiliegt und innerhalb der Fasern vergraben ist.

9. Knochenregenerationsmaterial nach Anspruch 8, wobei das freiliegende b-TCP Stellen zum Binden an das BMP-2 bereitstellt.

10. Knochenregenerationsmaterial nach Anspruch 8 oder Anspruch 9, wobei das freiliegende b-TCP Interaktion mit Osteoklasten und Osteoblasten während Remodellierung und Neuknochenbildung erleichtert.

11. Knochenregenerationsmaterial nach einem vorhergehenden Anspruch, wobei Lücken zwischen den biologisch abbaubaren Fasern 1 bis 1000 Mikrometer sind und ermöglichen, dass Körperfluid durch die Lücken dringt.

12. Knochenregenerationsmaterial nach einem der Ansprüche 1 bis 11, wobei die biologisch abbaubaren Fasern die mechanische Festigkeit aufweisen, um die Form der Fasern nach Implantation an der Knochenreparaturstelle beizubehalten.

13. Knochenregenerationsmaterial nach einem der Ansprüche 1 bis 12 zur Verwendung bei Knochenreparatur.

## Revendications

1. Matériau de régénération osseuse comprenant une structure de fibres biodégradables comprenant du phosphate de bêta-tricalcium (b-TCP) et un polymère biodégradable, et
une protéine-2 morphogénétique osseuse (BMP-2),
dans lequel la BMP-2 est liée au b-TCP,
dans lequel la BMP-2 est une protéine BMP-2 ciblable fusionnée avec un peptide de liaison au b-TCP, et la BMP-2 est liée au b-TCP par l'intermédiaire du peptide de liaison au b-TCP,
dans lequel le b-TCP représente 30 à 85 % en poids de la structure, et
dans lequel les fibres biodégradables comprennent des fibres dotées d'une épaisseur d'environ 5 micromètres à environ 100 micromètres, et les fibres biodégradables sont formées par électrofilage.

2. Matériau de régénération osseuse de la revendication 1, dans lequel le polymère biodégradable comprend de l'acide co-glycolique polylactique) (PLGA).

3. Matériau de régénération osseuse de la revendication 1 ou de la revendication 2, dans lequel la structure comprend un grand espace interstitiel pour permettre à des fluides biologiques de pénétrer facilement dans la structure.

4. Matériau de régénération osseuse d'une quelconque revendication précédente, dans lequel la structure comprend une grande aire de surface pour permettre la libération de calcium et de phosphore du b-TCP dans des fluides biologiques.

5. Matériau de régénération osseuse d'une quelconque revendication précédente, dans lequel la structure comprend une grande aire de surface pour supporter la liaison de BMP-2 à la structure.

6. Matériau de régénération osseuse d'une quelconque revendication précédente, dans lequel la structure est souple et peut être adaptée à la forme d'un site de réparation osseuse.

7. Matériau de régénération osseuse d'une quelconque revendication précédente, dans lequel le b-TCP est distribué dans les fibres.

8. Matériau de régénération osseuse d'une quelconque revendication précédente, dans lequel le b-TCP est exposé à la surface des fibres et enterré à l'intérieur des fibres.

9. Matériau de régénération osseuse de la revendication 8, dans lequel le b-TCP exposé fournit des sites de liaison à la BMP-2.

10. Matériau de régénération osseuse de la revendication 8 ou de la revendication 9, dans lequel le b-TCP exposé facilite l'interaction avec des ostéoclastes et des ostéoblastes pendant le remodelage et la formation de nouveaux os.

11. Matériau de régénération osseuse d'une quelconque revendication précédente, dans lequel des espaces entre les fibres biodégradables mesurent 1 à 1 000 micromètres, et permettent à du fluide corporel de pénétrer à travers les espaces.

12. Matériau de régénération osseuse de l'une quelconque des revendications 1 à 11, dans lequel les fibres biodégradables ont la résistance mécanique pour maintenir la forme des fibres après implantation au niveau du site de réparation osseuse.

13. Matériau de régénération osseuse de l'une quelconque des revendications 1 à 12, destiné à une utilisation dans la réparation osseuse.
